# EUROPEAN PATENT APPLICATION

(11) **EP 3 342 403 A1**
(43) Date of publication of application: **04.07.2018**
(21) Application number: 17211085.0
(22) Date of filing: 29.12.2017
(51) Int. Cl.: A61K 9/28, A61K 31/00

(54) **ORAL PHARMACEUTICAL COMPOSITIONS COMPRISING TADALAFIL AND DAPOXETINE**

(30) Priority: 30.12.2016 TR 201620151
(71) Applicant: Sanovel Ilac Sanayi ve Ticaret A.S., 34460 Istanbul (TR)
(72) Inventor: TÜRKYILMAZ, Ali, 34460 Istanbul (TR); GÜNER, Dicle, 34460 Istanbul (TR); YELKEN, Gülay, 34398 Istanbul (TR); PALANTÖKEN, Arzu, 34398 Istanbul (TR)
(74) Representative: Sevinç, Erkan

(57) **Abstract**

The present invention relates to pharmaceutical compositions comprising tadalafil or a pharmaceutically acceptable salt, solvate or polymorph thereof in combination with dapoxetine or a pharmaceutically acceptable salt, solvate or polymorph thereof.

## Description

### Field of Invention

The present invention relates to pharmaceutical compositions comprising tadalafil or a pharmaceutically acceptable salt, solvate or polymorph thereof in combination with dapoxetine or a pharmaceutically acceptable salt, solvate or polymorph thereof.

### Background of Invention

Selective serotonin reuptake inhibitors (SSRI) are used in the long-term prophylaxis of many types of depression, including the endogenous type, recurrent depression, and in the treatment of obsessive-compulsive disorders, panic attack, social phobias, and the bulimia nervosa disease. Dapoxetine, which is a selective serotonin reuptake inhibitor, is used for the treatment of depression and premature ejaculation. Its chemical name is (S)-N,N-Dimethyl-3-(naphthalen-1-yloxy)-1-phenylpropan-1-amine and it has a chemical structure shown in Formula I.

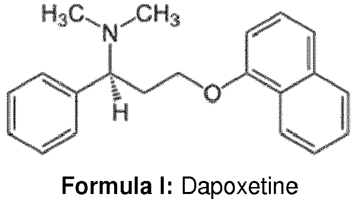

Orally-administered tablets of dapoxetine are commercially available under the name Priligy®. The most frequently encountered problem in oral dapoxetine formulations is the bitter taste. The tablets have typically been coated with coating agents, and mixtures of sweeteners or cation exchange resins have been used for masking the bitter taste.

On the other hand, phosphodiesterase type 5 inhibitors (PDE5 inhibitors) are used in the treatment of erectile dysfunction (ED). PDE5 inhibitors block the phosphodiesterase enzyme in a selective and efficient manner, thus increase the level of cyclic guanosine monophosphate (cGMP) in the corpus cavernosum smooth muscle cells. Most frequently used PDE5 inhibitors are avanafil, lodenafil, mirodenafil, sildenafil, tadalafil, vardenafil, and udenafil. Tadalafil, which is a PDE5 inhibitor used in the treatment of ED and Pulmonary Arterial Hypertension (PAH), has a longer half-life as compared to other PDE5 inhibitors. The chemical name of tadalafil is (6R-trans)-6-(1,3-benzodioxol-5-yl)-2,3,6,7,12,12a-hexahydro-2-methyl-pyrazino [1',2':1,6] pyrido[3,4-b]indole-1,4-dione, with the chemical structure illustrated below in Formula II.

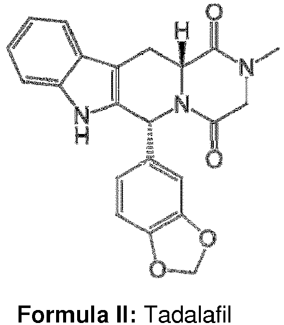

In the state of art, both dapoxetine comprising compositions and tadalafil comprising compositions have their own specific problems due to the active agents' undesirable physical properties or the selection of unsuitable excipients in the composition.

For instance, it is known that tadalafil is weakly soluble (practically insoluble) in water. Accordingly, it causes low dissolution rate in the gastrointestinal liquid, and consequently low bioavailability. Various methods have been performed to overcome this problem such as using surfactants and/or complexing agents and reducing the particle size of tadalafil.

On the side of dapoxetine, the leading technical problem is the short half-life (approximately 1.4 hours) and accordingly not last longing effect of the active agent. This disadvantage, which gives rise to necessity to repeat the administration of the drug, has been tolerated by formulating an extended release formulation. Another problem is failure to attain sufficient density while formulating the formulations comprising dapoxetine in tablet form and failure to obtain a formulation with good flow characteristics causing variations in weight and contents of the obtained tablets due to the low bulk density and poor flow characteristics of dapoxetine. In the prior art, this problem has been tried to be solved by the arrangement of the ratio of active agent to the diluent. Some other problems are the bitter taste and the solubility of dapoxetine which is dependent on the pH of the medium; namely it is soluble in acid, however it is practically insoluble at neutral pH.

In the case of composing a combination thereof, it is obvious that these problems shall increasingly continue. Nevertheless, it is still advisable to benefit from the synergetic effect of the combination of these active agents providing correlatively supportive indications.

It is known that combination therapy reduces the daily dosages to be taken by patients and simplifies dosing schedule thereby increases patient compliance and the drug efficacy. Thus, there are various combinations of PDE5 inhibitors and selective serotonin reuptake inhibitors in the state of art.

In the patent application numbered WO2011087755 A2 a method to treat distress dysfunction conditions, symptoms and/or disorders is revealed and a composition comprising PDE inhibitors and selective serotonin reuptake inhibitors other than various classes of drug is suggested. This application aims to bring an approach to the treatment of the aforementioned disorders; however, it does not suggest any established formulation, based on dapoxetine and tadalafil, which provides the required stability and bioavailability.

Similarly, the patent document EP1671628 B1 presents the use of dapoxetine and a phosphodiesterase inhibitor for the preparation of a medicament to treat sexual dysfunction in a mammal.

In the state of art, even if it's insufficient, there are also dapoxetine and tadalafil combinations which are formulated as having bilayer tablet form to provide the controlled-release of the drug. These compositions inevitably comprise hydroxypropyl methycellulose, which is a controlled-release agent, to ensure the required medical effect.

It is clear that there is no teaching, suggestion or motivation in the prior art about how to develop a stable immediate-release composition for oral administration comprising dapoxetine and tadalafil. Accordingly, considering the above mentioned examples, there is still a need for a solid oral formulation comprising this combination which is free of hydroxypropyl methylcellulose and which eliminates especially the low solubility problem of tadalafil and the short half-life problem of dapoxetine to enhance the dissolution profile and bioavailability of the final pharmaceutical product.

### Objects and Brief Description of the Invention

The main object of the present invention is to obtain combination formulations, comprising dapoxetine or a pharmaceutically acceptable salt, solvate or polymorph thereof in combination with tadalafil or a pharmaceutically acceptable salt, solvate or polymorph thereof, eliminating all aforesaid problems and bringing additional advantages to the relevant prior art.

Another object of the present invention is to obtain combination formulations of dapoxetine and tadalafil with high stability and bioavailability.

A further object of the present invention is to develop combination formulations of dapoxetine and tadalafil having an improved level of dissolution rate and solubility.

Another object of the present invention is to provide a tablet formulation of dapoxetine and tadalafil which is free of hydroxypropyl methylcellulose.

Yet, another object of the present invention is to provide a dapoxetine and tadalafil tablet comprising at least one film coating to protect the pharmaceutical composition against the moisture and light to maintain the stability.

A further object of the present invention is to improve a process for preparing the said coated tablet comprising wet granulation and coating.

### Detailed Description of Invention

In accordance with the objects outlined above, detailed features of the present invention are given herein.

The present invention relates to an oral pharmaceutical compositions comprising dapoxetine or a pharmaceutically acceptable salt, solvate or polymorph thereof in combination with tadalafil or a pharmaceutically acceptable salt, solvate or polymorph.

According to the preferred embodiment of the invention, the composition is essentially free of hydroxypropyl methylcellulose, since it reduces the dissolution rate and solubility.

According to one preferred embodiment, the composition subjected to the invention is also free of dibasic calcium phosphate, since it is irritating to the gastrointestinal tract and creates at least 1% by weight of lubricant requirement in the solid oral composition.

According to the preferred embodiment of the invention, the active agents used in the composition are dapoxetine hydrochloride (dapoxetine HCI) and tadalafil.

Particle size distributions of these active agents play a significant role for the qualification of the composition subjected to the invention. As used herein, 'particle size distribution' means the cumulative volume size distribution as tested by any conventionally accepted method such as the laser diffraction method (Malvern analysis).

Laser diffraction measures particle size distributions by measuring the angular variation in intensity of light scattered as a laser beam passes through a dispersed particulate sample. Large particles scatter light at small angles relative to the laser beam and small particles scatter light at large angles. The angular scattering intensity data is then analyzed to calculate the size of the particles responsible for creating the scattering. The particle size is reported as a volume equivalent sphere diameter.

According to this measuring method, the D50 value is the size in microns that splits the distribution with half above and half below this diameter. Similarly, 90% of the distribution lies below the D90 value, and 10% of the distribution lies below the D10 value.

The D50 value of the particles of dapoxetine or a pharmaceutically acceptable salt, solvate or polymorph thereof is ranging between 1 to 200 µm. Preferably, this value is lower than 50 µm.

The D90 value of the particles of dapoxetine or a pharmaceutically acceptable salt, solvate or polymorph thereof is ranging between 1 to 500 µm. Preferably, this value is lower than 250 µm.

The D10 value of the particles of dapoxetine or a pharmaceutically acceptable salt, solvate or polymorph thereof is lower than 10 µm.

The D50 value of the particles of tadalafil or a pharmaceutically acceptable salt, solvate or polymorph thereof is ranging between 1 to 100 µm. Preferably, this value is lower than 30 µm.

The D90 value of the particles of tadalafil or a pharmaceutically acceptable salt, solvate or polymorph thereof is ranging between 1 to 300 µm. Preferably, this value is lower than 100 µm.

The D10 value of the particles of tadalafil or a pharmaceutically acceptable salt, solvate or polymorph thereof is lower than 10 µm.

These specific selections of particle size distribution values, especially the D50 value range of tadalafil or a pharmaceutically acceptable salt, solvate or polymorph thereof, surprisingly increase the solubility of the composition in the gastric medium and enhance the dissolution rate.

In the preferred embodiment of the invention, the ratio of the D50 value of dapoxetine or a pharmaceutically acceptable salt, solvate or polymorph thereof to the D50 value of tadalafil or a pharmaceutically acceptable salt, solvate or polymorph thereof is in the range of 10:1 to 0.1:1, preferably 5:1 to 0.5:1 and more preferably 4:1 to 1:1.

It has been seen that this specific range promotes the tablet compression process.

According to this preferred embodiment, the amount of dapoxetine is between 1-60% by weight of the total composition. Preferably this amount is between 5-40% by weight of the total composition. More preferably dapoxetine is present between 10-20% by weight in the total composition.

According to this preferred embodiment, the amount of tadalafil is between 1-30% by weight of the total composition. Preferably this amount is between 1-20% by weight of the total composition. More preferably tadalafil is present between 3-15% by weight in the total composition.

Dapoxetine is present in an amount of 1 to 300 mg, preferably 10 to 200 mg and more preferably 20 to 100 mg in the composition.

According to the one of the most preferred embodiment of the invention, the amount of dapoxetine HCI is 67.2 mg which is equal to 60 mg dapoxetine.

According to the another most preferred embodiment of the invention, the amount of dapoxetine HCI is 33,6 mg which is equal to 30 mg dapoxetine.

On the other hand, tadalafil is present in an amount of 1 to 200 mg, preferably 5 to 100 mg and more preferably 10 to 50 mg.

According to the most preferred embodiment of the invention, the amount of tadalafil is 20 mg.

In the preferred embodiment of the invention, the ratio of dapoxetine to tadalafil is in the range of 10:1 to 0.1:1 and preferably 5:1 to 0.5:1, more preferably 4:1 to 1:1 by weight. According to the most preferred embodiments, this ratio is 3:1 or 1.5:1. This preferred selection of range assures the enhanced bioavailability during the shelf life.

According to these embodiments, the composition is in the form of coated tablet, trilayer tablet, bilayer tablet, multilayer tablet, orally disintegrating tablet, mini tablet, pellet, sugar pellet, syrup, suspension, elixir, buccal tablet, sublingual tablet, effervescent tablet, immediate release tablet, modified release tablet, film-coated tablet, gastric disintegrating tablet, pill, capsule, oral granule, powder, coated bead system, microsphere, tablet in tablet, inlay tablet, dragee, sachet or orally administrable film.

The composition is preferably in the form of a film-coated tablet.

According to this embodiment, the composition comprises at least one pharmaceutically acceptable excipient selected from binders, diluents, disintegrants, lubricants, glidants, surfactants, coating materials or mixtures thereof.

According to one embodiment of the invention, the oral pharmaceutical composition comprises at least one diluent which is selected from the group comprising lactose monohydrate, lactose, microcrystalline cellulose, mannitol, spray-dried mannitol, starch, dextrose, sucrose, fructose, maltose, sorbitol, xylitol, inositol, kaolin, inorganic salts, polysaccharides, sodium chloride, dextrates, lactitol, maltodextrin, sucrose-maltodextrin mixture, trehalose, sodium carbonate, sodium bicarbonate, calcium carbonate or mixtures thereof.

According to the preferred embodiment of the invention, the oral pharmaceutical composition comprises three diluents which are fine mannitol, coarse mannitol and microcrystalline cellulose.

Fine mannitol is the mannitol powder with the D50 value ranging between 1-180 µm, preferably lower than 60 µm. Preferably the fine mannitol is mannitol 60.

Coarse mannitol is the mannitol powder with the D50 value ranging between 20-600 µm, preferably lower than 400 µm. Preferably the coarse mannitol is mannitol DC 400.

The amount of microcrystalline cellulose is between 5-50%, preferably 10-40% and more preferably 15-30% by weight of the total composition.

The amount of fine mannitol is between 1-50%, preferably 5-30% and more preferably 10-25% by weight of the total composition.

The amount of coarse mannitol is between 1-50%, preferably 5-30% and more preferably 10-25% by weight of the total composition.

In the preferred embodiment of the invention, the ratio of fine mannitol to coarse mannitol is in the range of 1:0.1 to 5:1, preferably 1:0.2 to 4:1 and more preferably 1:0.5 to 1:2 by weight. This preferred selection of range assures the enhanced stability during the shelf life.

According to one embodiment of the invention, the oral pharmaceutical composition comprises at least one binder which are selected from the group comprising polyvinylpyrrolidone (PVP, povidone), microcrystalline cellulose, copovidone, copolyvidone, polyvinylpyrrolidone K30 (povidone K30), carnauba wax, pullulan, polymethacrylate, glyceryl behenate, hydroxypropyl cellulose (HPC), carboxymethyl cellulose (CMC), methyl cellulose (MC), hydroxyethyl cellulose, sodium carboxymethyl cellulose (Na CMC), ethyl cellulose, polymetacrylates, polyethylene oxide, polyvinyl alcohol, polycarbophil, polyvinyl acetate and its copolymers, gelatin, xanthan gum, guar gum, alginate, carrageen, kollagen, agar, pectin, hyaluronic acid, carbomer, cellulose acetate phthalate, hydroxyethyl methyl cellulose, polaxomer, polyethylene glycol (PEG), sugars, glycose syrups, natural gums, tragacanth gum, polyacrylamide, aluminum hydroxide, benthonite, laponite, setostearyl alcohol, polyoxyethylene-alkyl ethers, acacia mucilage, polydextrose or mixtures thereof.

According to the preferred embodiment of the invention, the oral pharmaceutical composition comprises one binder which is polyvinylpyrrolidone.

The amount of polyvinylpyrrolidone is between 0.1-5%, preferably 0.5-2% by weight of the total composition.

According to one embodiment of the invention, the oral pharmaceutical composition comprises at least one disintegrant which is selected from the group comprising croscarmellose sodium, sodium starch glycolate, sodium carbonate, hydroxylpropyl cellulose (HPC), cross-linked polyvinylpyrrolidone (crospovidone), copovidon, polycarbophil, low-substitue poloxamer, alginic acid and alginates, ion-exchange resins, magnesium aluminum silica, sodium carboxy methyl cellulose, carboxy methyl cellulose calcium, docusate sodium, guar gum, polyacrylin potasium, sodium alginate, sodium glycine carbonate or mixtures thereof.

According to the preferred embodiment of the invention, the oral pharmaceutical composition comprises one disintegrant which is crospovidone. Preferably, the D50 value of crospovidone is lower than 50 µm.

The amount of crospovidone is between 1-50%, preferably 5-30% and more preferably 10-20% by weight of the total composition.

Preferably, crospovidone, used in the composition, is Kollidon® CL-SF.

In the preferred embodiment of the invention, the ratio of the total weight of disintegrant to the total weight of diluent is in the range of 1:1 to 1:8, preferably 1:2 to 1:6 and more preferably 1:3 to 1:5.

According this preferred embodiment of the invention, the ratio of the total weight of disintegrant to the total weight of binder is in the range of 1:1 to 30:1, preferably 5:1 to 25:1 and more preferably 10:1 to 20:1.

These preferred selection of range assures the improved dissolution and disintegration profiles.

According to one embodiment of the invention, the oral pharmaceutical composition comprises at least one lubricant, at least one glidant and at least one surfactant which are selected from the group comprising sodium lauryl sulfate, sodium stearyl fumarate, magnesium stearate, colloidal silicon dioxide, zinc stearate, calcium stearate, mineral oil, talc, polyethylene glycol, glyceryl monostearate, glyceryl palmitostearate, magnesium lauryl sulfate, fumaric acid, zinc stearate, stearic acid, hydrogenated natural oils, silica, paraffin or mixtures thereof.

According to the preferred embodiment of the invention, the oral pharmaceutical composition comprises one lubricant which is magnesium stearate.

The amount of magnesium stearate is between 0.1-5%, preferably 0.2-1% by weight of the total composition.

According to the preferred embodiment of the invention, the oral pharmaceutical composition comprises one glidant which is colloidal silicon dioxide.

The amount of colloidal silicon dioxide is between 0.1-5%, preferably 0.4-1% by weight of the total composition.

According to the preferred embodiment of the invention, the oral pharmaceutical composition comprises one surfactant which is sodium lauryl sulfate.

The amount of sodium lauryl sulfate is between 0.1-5%, preferably 0.3-1% by weight of the total composition.

The existence of colloidal silicon dioxide and sodium lauryl sulfate in these preferred ranges enhances the wettability and disintegration rate of the solid oral composition subjected to the invention.

According to one embodiment of the invention, the solid oral pharmaceutical composition comprises at least one coating layer to protect the composition against the moisture and light to maintain the stability. Suitable coating ingredients are selected from the group comprising hypromellose, lactose monohydrate, hydroxypropyl cellulose, polyvinyl alcohol (PVA), polyethylene glycol (PEG), talc, polyvinyl alcohol-polyethylene glycol copolymers (Kollicoat® IR), ethylcellulose dispersions (Surelease®), polyvinylprolidone, polyvinylprolidone-vinyl acetate copolymer (PVP-VA), all kinds of Opadry®, pigments, dyes, titanium dioxide, iron oxide or mixtures thereof.

According to one embodiment, coating layer is Opadry II Blue which comprises polyvinyl alcohol, polyethylene glycol, titanium dioxide, talc, blue pigment (FD&C blue no.2) and quinoline yellow (aluminium lake).

According to one preferred embodiment, the oral pharmaceutical composition is in the form of film-coated tablet comprising dapoxetine hydrochloride and tadalafil as active agents, fine mannitol (which is mannitol 60), coarse mannitol (which is mannitol DC 400) and microcrystalline cellulose as diluents, polyvinylpyrrolidone as binder, crospovidone as disintegrant, magnesium stearate as lubricant, colloidal silicon dioxide as glidant and sodium lauryl sulphate as surfactant and a coating layer.

According to this preferred embodiment, the composition comprises;
- 1-60% by weight dapoxetine hydrochloride,
- 1-30% by weight of tadalafil,
- 1-50% by weight of fine mannitol,
- 1-50% by weight of coarse mannitol,
- 5-50% by weight of microcrystalline cellulose,
- 0.1-5% by weight of polyvinylpyrrolidone,
- 1-50% by weight of crospovidone,
- 0.1-5% by weight of magnesium stearate,
- 0.1-5% by weight of colloidal silicon dioxide,
- 0.1-5% by weight of sodium lauryl sulfate,
- 1-5% by weight of moisture protective coating.

These analytically selected ratios ensure the required effective doses for the treatment and enhance the stability and dissolution profile of the film-coated tablet subjected to the invention.

According to all these embodiments, the below given formulations can be used in the solid oral pharmaceutical composition subjected to the invention. These examples are not limiting the scope of the present invention and should be considered under the light of the foregoing detailed disclosure.

### Example 1: Film-coated tablet

| **Ingredients** | **Amount (%)** |
|---|---|
| Dapoxetine hydrochloride | 10 - 20 |
| Tadalafil | 3 - 15 |
| Mannitol 60 (fine mannitol) | 10 - 25 |
| Mannitol DC 400 (coarse mannitol) | 10 - 25 |
| Microcrystalline cellulose (Avicel® PH 102) | 15 - 30 |
| Polyvinylpyrrolidone K30 | 0.5 - 2 |
| Crospovidone (Kollidon® CL-SF) | 10 - 20 |
| Magnesium stearate | 0.2 - 1 |
| Colloidal silicon dioxide | 0.4 - 1 |
| Sodium lauryl sulfate | 0.3 - 1 |
| **Total tablet** | **100** |
| Coating | 1 - 5 |

| **Coating Material (Opadry II Blue) Ingredients** | **Amount (%)** |
|---|---|
| Polyvinyl alcohol (PVA) | 20 - 60 |
| Polyethylene glycol (PEG) | 10 - 30 |
| Titanium dioxide | 10 - 30 |
| Talc | 10 - 20 |
| Blue pigment (FD&C blue no.2) | 2 - 8 |
| Quinoline yellow (aluminium lake) | 0.1 - 0.5 |
| **Total coating** | **100** |

### Example 2: Film-coated tablet

| **Ingredients** | **Amount (%)** |
|---|---|
| Dapoxetine hydrochloride | 16.8 |
| Tadalafil | 5.0 |
| Mannitol 60 (fine mannitol) | 12.5 |
| Mannitol DC 400 (coarse mannitol) | 22.5 |
| Microcrystalline cellulose (Avicel® PH 102) | 25.4 |
| Polyvinylpyrrolidone K30 | 1.0 |
| Crospovidone (Kollidon® CL-SF) | 15.0 |
| Magnesium stearate | 0.5 |
| Colloidal silicon dioxide | 0.7 |
| Sodium lauryl sulfate | 0.6 |

| **Total tablet** | **100.0** |
|---|---|
| Coating | 3.0 |

| **Coating Material (Opadry II Blue) Ingredients** | **Amount (%)** |
|---|---|
| Polyvinyl alcohol (PVA) | 40.0 |
| Polyethylene glycol (PEG) | 20.2 |
| Titanium dioxide | 19.1 |
| Talc | 14.8 |
| Blue pigment (FD&C blue no.2) | 5.5 |
| Quinoline yellow (aluminium lake) | 0.4 |
| **Total coating** | **100.0** |

| | |
|---|---|
| *16.8% by weight of dapoxetine hydrochloride is equivalent to 15% by weight of dapoxetine | |

### Example 3: Film-coated tablet

| **Ingredients** | **Amount (%)** |
|---|---|
| Dapoxetine hydrochloride | 16.8 |
| Tadalafil | 10.0 |
| Mannitol 60 (fine mannitol) | 20.0 |
| Mannitol DC 400 (coarse mannitol) | 15.0 |
| Microcrystalline cellulose (Avicel® PH 102) | 19.8 |
| Polyvinylpyrrolidone K30 | 1.0 |
| Crospovidone (Kollidon® CL-SF) | 15.0 |
| Magnesium stearate | 0.5 |
| Colloidal silicon dioxide | 0.7 |
| Sodium lauryl sulfate | 1.2 |
| **Total tablet** | **100.0** |
| Coating | 3.0 |

| **Coating Material (Opadry II Blue) Ingredients** | **Amount (%)** |
|---|---|
| Polyvinyl alcohol (PVA) | 40.0 |
| Polyethylene glycol (PEG) | 20.2 |
| Titanium dioxide | 19.1 |
| Talc | 14.8 |
| Blue pigment (FD&C blue no.2) | 5.5 |
| Quinoline yellow (aluminium lake) | 0.4 |
| **Total coating** | **100.0** |

| | |
|---|---|
| *16.8% by weight of dapoxetine hydrochloride is equivalent to 15% by weight of dapoxetine | |

The pharmaceutical formulations mentioned above are prepared by following these steps:
- mixing fine mannitol, two-thirds of crospovidone and tadalafil together for about 10 minutes to prepare a powder mixture comprising tadalafil
- on the other side, solving sodium lauryl sulfate and polyvinylpyrrolidone K30 in water to prepare a homogenous granulation solution
- granulating the powder mixture comprising tadalafil with the granulation solution
- adding water if needed during the granulation procedure
- drying and sieving the granules comprising tadalafil
- mixing this dried and sieved mixture with one-third of crospovidone, microcrystalline cellulose, coarse mannitol, dapoxetine hydrochloride and colloidal silicon dioxide for about 15 minutes
- adding magnesium stearate and mixing the total mixture
- compressing the total mixture into tablets
- preparing an aqueous solution of coating material and coating the tablets with this solution in a way to form a film layer

## Claims

1. An oral pharmaceutical composition comprising dapoxetine or a pharmaceutically acceptable salt, solvate or polymorph thereof and tadalafil or a pharmaceutically acceptable salt, solvate or polymorph thereof; wherein the composition is substantially free of hydroxypropyl methylcellulose.

2. An oral pharmaceutical composition comprising dapoxetine or a pharmaceutically acceptable salt, solvate or polymorph thereof and tadalafil or a pharmaceutically acceptable salt, solvate or polymorph thereof; wherein the D50 value of the particles of tadalafil or a pharmaceutically acceptable salt, solvate or polymorph thereof is ranging between 1 to 100 µm.

3. The oral pharmaceutical composition according to claim 1 or 2, wherein the said dapoxetine salt is dapoxetine hydrochloride.

4. The oral pharmaceutical composition according to any preceding claim, wherein the D50 value of the particles of dapoxetine or a pharmaceutically acceptable salt, solvate or polymorph thereof is ranging between 1 to 200 µm, preferably it is lower than 50 µm.

5. The oral pharmaceutical composition according to any preceding claim, wherein the D50 value of the particles of tadalafil or a pharmaceutically acceptable salt, solvate or polymorph thereof is lower than 30 µm.

6. The oral pharmaceutical composition according to claim 5, wherein the ratio of the D50 value of dapoxetine or a pharmaceutically acceptable salt, solvate or polymorph thereof to the D50 value of tadalafil or a pharmaceutically acceptable salt, solvate or polymorph thereof is in the range of 10:1 to 0.1:1, preferably 5:1 to 0.5:1 and more preferably 4:1 to 1:1.

7. The oral pharmaceutical composition according to any one of claims 1 to 3, wherein the D90 value of the particles of dapoxetine or a pharmaceutically acceptable salt, solvate or polymorph thereof is ranging between 1 to 500 µm, preferably it is lower than 250 µm.

8. The oral pharmaceutical composition according to any one of claims 1 to 3, wherein the D10 value of the particles of dapoxetine or a pharmaceutically acceptable salt, solvate or polymorph thereof is lower than 10 µm.

9. The oral pharmaceutical composition according to claim 1 or 2, wherein the D90 value of the particles of tadalafil or a pharmaceutically acceptable salt, solvate or polymorph thereof is ranging between 1 to 300 µm, preferably it is lower than 100 µm.

10. The oral pharmaceutical composition according to claim 1 or 2, wherein the D10 value of the particles of tadalafil or a pharmaceutically acceptable salt, solvate or polymorph thereof is lower than 10 µm.

11. The oral pharmaceutical composition according to any one of claims 1 to 3, wherein the composition comprises dapoxetine in an amount of 1-60%, preferably 5-40% and more preferably 10-20% by weight.

12. The oral pharmaceutical composition according to claim 1 or 2, wherein the composition comprises tadalafil in an amount of 1-30%, preferably 1-20% and more preferably 3-15% by weight.

13. The oral pharmaceutical composition according to any preceding claims, wherein the composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, film-coated tablet, gastric disintegrating tablet, pill, capsule, oral granule, powder, coated bead system, microsphere, tablet in tablet, inlay tablet, dragee, sachet or orally administrable film, preferably the composition is in the form of a film-coated tablet.

14. The oral pharmaceutical composition according to any preceding claims, wherein the composition further comprises at least one pharmaceutically acceptable excipient selected from binders, diluents, disintegrants, lubricants, glidants, surfactants, coating materials or mixtures thereof.

15. The oral pharmaceutical composition according to claim 14, wherein the composition comprises three diluents which are fine mannitol whose D50 value is ranging between 1-180 µm, coarse mannitol whose D50 value ranging between 20-600 µm and microcrystalline cellulose.

16. The oral pharmaceutical composition according to claim 15, wherein the ratio of fine mannitol to coarse mannitol is in the range of 1:0.1 to 5:1, preferably 1:0.2 to 4:1 and more preferably 1:0.5 to 1:2 by weight.

17. The oral pharmaceutical composition according to claim 14, wherein the composition comprises one disintegrant which is crospovidone whose D50 value is lower than 50 µm.

18. The oral pharmaceutical composition according to claim 14, wherein the ratio of the total weight of disintegrant to the total weight of diluent is in the range of 1:1 to 1:8, preferably 1:2 to 1:6 and more preferably 1:3 to 1:5.

19. The oral pharmaceutical composition according to any preceding claims, wherein the composition comprises;
- 1-60% by weight dapoxetine hydrochloride,
- 1-30% by weight of tadalafil,
- 1-50% by weight of fine mannitol,
- 1-50% by weight of coarse mannitol,
- 5-50% by weight of microcrystalline cellulose,
- 0.1-5% by weight of polyvinylpyrrolidone,
- 1-50% by weight of crospovidone,
- 0.1-5% by weight of magnesium stearate,
- 0.1-5% by weight of colloidal silicon dioxide,
- 0.1-5% by weight of sodium lauryl sulfate,
- 1-5% by weight of moisture protective coating.

20. A process for preparing the oral pharmaceutical composition according to claim 19, comprising the following steps:
- mixing fine mannitol, two-thirds of crospovidone and tadalafil together to prepare a powder mixture comprising tadalafil
- on the other side, solving sodium lauryl sulphate and polyvinylpyrrolidone in water to prepare a homogenous granulation solution
- granulating the powder mixture comprising tadalafil with the granulation solution
- adding water if needed during the granulation procedure
- drying and sieving the granules comprising tadalafil
- mixing this dried and sieved mixture with one-third of crospovidone, microcrystalline cellulose, coarse mannitol, dapoxetine hydrochloride and colloidal silicon dioxide
- adding magnesium stearate and mixing the total mixture
- compressing the total mixture into tablets
- preparing an aqueous solution of coating material and coating the tablets with this solution in a way to form a film layer
